(19) Europäisches Patentamt
European Patent Office
Office européen des brevets

(11) **EP 4 362 780 B1**

(12) **EUROPEAN PATENT SPECIFICATION**

(45) Date of publication and mention
of the grant of the patent:
**04.03.2026 Bulletin 2026/10**

(21) Application number: **22834322.4**

(22) Date of filing: **01.07.2022**

(51) International Patent Classification (IPC):
**A61B 5/0205** (2006.01)   **A61B 5/024** (2006.01)
**G16H 10/60** (2018.01)   A61B 5/00 (2006.01)

(52) Cooperative Patent Classification (CPC):
**A61B 5/02055; A61B 5/7282; A61B 5/746;**
**G16H 50/30;** A61B 5/4866; A61B 2562/0271

(86) International application number:
**PCT/US2022/035990**

(87) International publication number:
**WO 2023/278858 (05.01.2023 Gazette 2023/01)**

(54) **THERMOREGULATORY STRESS DETECTION FROM SKIN TEMPERATURE COMPLEXITY**

THERMOREGULIERENDE STRESSDETEKTION AUS DER HAUTTEMPERATURKOMPLEXITÄT

DÉTECTION DE STRESS THERMORÉGULATEUR À PARTIR DE LA COMPLEXITÉ DE LA TEMPÉRATURE DE LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.07.2021 US 202163217600 P**

(43) Date of publication of application:
**08.05.2024 Bulletin 2024/19**

(73) Proprietors:
• **The Government of the United States, as represented by the Secretary of the Army**
Fort Detrick, Maryland 21702-9223 (US)
• **The Secretary of State for Defence of the United Kingdom of Great Britain and Northern Ireland**
London Greater London SW1A 2HB (GB)

(72) Inventors:
• **BULLER, Mark Jonathan**
Natick, Massachusetts 01760-5007 (US)
• **DELVES, Simon K.**
Alverstoke Hampshire PO12 2DL (GB)

(74) Representative: **Gardner, Emma Elizabeth**
**Defence Intellectual Property Rights**
**Poplar 2-2214**
**MOD Abbey Wood (South)**
**Bristol BS34 8JH (GB)**

(56) References cited:
WO-A2-2020/180454   US-A1- 2007 198 072
US-A1- 2017 249 445   US-A1- 2019 387 982
US-A1- 2019 387 982   US-A1- 2021 106 238

• MARK J BULLER ET AL: "NOTE; A real-time heat strain risk classifier using heart rate and skin temperature", PHYSIOLOGICAL MEASUREMENT, INSTITUTE OF PHYSICS PUBLISHING, BRISTOL, GB, vol. 29, no. 12, 1 December 2008 (2008-12-01), pages N79 - N85, XP020145335, ISSN: 0967-3334
• DAVEY SARAH L ET AL: "The physiological strain index does not reliably identify individuals at risk of reaching a thermal tolerance limit", EUROPEAN JOURNAL OF APPLIED PHYSIOLOGY, SPRINGER VERLAG, HEIDELBERG, DE, vol. 121, no. 6, 7 March 2021 (2021-03-07), pages 1701 - 1713, XP037462898, ISSN: 1439-6319, [retrieved on 20210307], DOI: 10.1007/S00421-021-04642-3

**(Cont. next page)**

• BULLER MARK J ET AL: "REVIEW Emerging Wearable Physiological Monitoring Technologies & Decision Aids for Health & Performance Wearable physiological monitoring for human thermal-work strain optimization", J APPL PHYSIOL, 23 February 2018 (2018-02-23), pages 432 - 441, XP055824596, Retrieved from the Internet <URL:https://journals.physiology.org/doi/pdf/10.1152/japplphysiol.00353.2017> [retrieved on 20210715]
• BULLER MARK J ET AL: "Estimated and measured core temperature responses to high-intensity warm weather military training: implications for exertional heat illness risk assessment", PHYSIOLOGICAL MEASUREMENT., vol. 41, no. 6, 1 June 2020 (2020-06-01), GB, pages 65011, XP093299231, ISSN: 0967-3334, DOI: 10.1088/1361-6579/ab934b
• DAVEY SARAH L.; DOWNIE VICTORIA; GRIGGS KATY; HAVENITH GEORGE: "The physiological strain index does not reliably identify individuals at risk of reaching a thermal tolerance limit", EUROPEAN JOURNAL OF APPLIED PHYSIOLOGY, SPRINGER VERLAG, HEIDELBERG, DE, vol. 121, no. 6, 7 March 2021 (2021-03-07), DE
, pages 1701 - 1713, XP037462898, ISSN: 1439-6319, DOI: 10.1007/s00421-021-04642-3
• POKORA ILONA, ŻEBROWSKA ALEKSANDRA: "Application of A Physiological Strain Index in Evaluating Responses to Exercise Stress – A Comparison Between Endurance and High Intensity Intermittent Trained Athletes", JOURNAL OF HUMAN KINETICS, vol. 50, no. 1, 1 April 2016 (2016-04-01), pages 103 - 114, XP093022042, ISSN: 1640-5544, DOI: 10.1515/hukin-2015-0142

**Description**

This application claims the benefit and priority to U.S. Pat. App. No. 63/217,600 filed on July 1, 2021.

I. **Field of the** Disclosure

The disclosure in at least one embodiment relates to a system and/or a method for detecting when an individual may be under and/or about to be under thermoregulatory stress based, in part, on skin temperature complexity. An example system includes a heart rate sensor and a skin temperature sensor providing physiological signals to a processor. In a more particular embodiment, the disclosure provides a thermoregulatory strain index (TSI) or TSI score for the individual based on the skin temperature signal complexity and the heart rate complexity (or, alternatively, the mean heart rate) over a predetermined measurement window, which in at least one embodiment is adjustable. In a further embodiment, the TSI uses a difference between the resting complexity (or an approximation) and the current complexity for the skin temperature and/or the heart rate. In at least one embodiment, TSI uses a ratio of the skin temperature complexity to the heart rate complexity or *vice versa* for the individual, which in a further embodiment may be normalized for an individual using the individual's resting skin temperature and heart rate complexities, and in a further embodiment using maximum and minimum complexities across a particular population.

II. **Background** of the Invention

Hot environments pose a risk of heat illness to athletes and people in occupations where heavy workloads and/or protective clothing ensembles are necessary. Excessive heat strain can lead to collapse or even death. Heat-related illness (HRI) has a spectrum of disorders due to environmental heat exposure and includes minor conditions such as heat rash, heat cramps, heat syncope, rhabdomyolysis, and heat exhaustion as well as the more severe condition known as heat stroke. The treatment of HRIs can lead to unnecessary and expensive medical care, loss of work or training days, lower effectiveness and readiness of employment staff, and impact on the individual that suffers the HRI.

HRI is an ever-present threat to athletes, military personnel, and occupational hazard workers, as the combination of physical exertion in hot environments makes individuals susceptible to heat stroke, heat exhaustion, and heat cramps. HRI prevention includes avoiding medications that can increase the risk of heat illness (e.g., antihypertensives, diuretics, and anticholinergics), gradual adjustment to heat, and sufficient fluids and electrolytes. Mild HRI can be treated by drinking fluids. In more significant HRI cases, spraying with mist and using a fan is useful. For those with severe HRI putting them in lukewarm to cold water is recommended, if possible, with transport to a hospital.

HRI incidence risk is heightened when performing hard physical labor, or when weather or clothing impair heat loss. In 2018, there were 578 reported cases of heat stroke (0.45 cases per 1000) and 2,214 cases of heat exhaustion (1.71 cases per 1000) in the US military (Medical Surveillance Monthly Report (2019)). Between 2016-2020, there were over 12,484 HRI events at US military facilities with 2,615 of these cases being exertional heat strokes of which 475 exertional heat strokes were in 2020 (Heat Illness, Active Component, U.S. Armed Forces, 2020). One mitigation strategy is to modify training intensity or clothing to reduce the chance of excessive thermal strain; however, this is not always possible. As such it would be beneficial to identify individuals with impending heat illness early.

Ingestible pills that sense temperature are available to measure internal body temperature in real-time. However, they remain relatively expensive for day-to-day monitoring and administratively burdensome, particularly in situations where large groups are participating in an event and are to be monitored. The Estimated Core Temperature (ECTemp™) algorithm introduced by Buller *et al.* (2013) predicts body core temperature from heart rate alone, and it has been validated under a variety of conditions from temperate to hot climates, different clothing configurations (shorts and t-shirt to full personal protective equipment encapsulation (Buller *et al.* 2015)), and work intensities ranging from rest (Sim *et al.* 2016) to greater than 650 W (Buller et al. 2013).

Efforts to identify and control the incidence of heat illness/injury originally focused on identifying high risk environments and modifying work/rest schedules. Although the risk of HRI can be reduced by acclimation, appropriate work rest schedules, and proper hydration, the risk is never entirely abated when there is heavy exertion in a hot environment.

Assessing risk of heat stress from environmental conditions alone fails to account for individual differences, such as acclimation status, fitness, body composition and morphology, prior heat injury, which can play a role in an individual's response to working in hot environments and clothing.

Personal physiological monitoring is one means of overcoming the limitations of assessing thermal strain using environment monitoring alone. Modern physiological monitoring systems are becoming more common for monitoring applications; however, current measures or indices of heat strain (e.g., Physiological Strain Index, adaptive Physiological Strain Index, estimates of core temperature monitoring etc.) track well to overall level of strain but only provide a measure of general risk to succumbing to an HRI. While, this is useful it does not provide a specific risk of an individual succumbing to an HRI. What is needed is an index or measure of how well or not an individual is coping with a given heat strain and/or have an earlier indication that the individual may succumb to an HRI with time to prevent and/or lessen the risk of long-term effect of an HRI on the individual.

Skin temperature (Tsk) measures, along with core temperature (Tcr), and heart rate (HR) are all interrelated in the human thermoregulatory system. ECTemp core temperature estimates make use of the dual information in the heart rate of the cardiac output supplying fuel and oxygen to the muscles and the volume of blood used for heat transfer through vasodilation (Buller 2015). Similarly, the gradient between skin temperature and core temperature can provide an overall indication of the overall cardiac output (Sawka and Young 2006), and has been used in an adaptive physiological strain index (aPSI) (Buller, PCT Pub. No. WO 2017/181195 A1). In many instances of exercising in the heat skin blood flow and sweating are a major component of heat loss. Under extreme conditions of exercise and heat there can be conflicting demands for physiological resources and these may be manifested in the thermoregulatory control mechanism of skin blood flow. Previous work has utilized complexity measures to examine the state of control mechanisms. A change in heart rate variability has long been viewed as a tool to indicate a pathological change in an underlying control mechanism that correlates to a disease pathology (Shaffer F & Ginsberg JP (2017) and Ahmad et al. (2009)). Similarly, complexity of the core temperature and skin temperature relationship has been examined as a means to predict mortality and fever in patients (Varela et al. 2009). These considerations are also explored in US 2019/387982 A1 and Buller Mark J ET AL: "Estimated and measured core temperature responses to high-intensity warm weather military training: implications for exertional heat illness risk assessment", PHYSIOLOGICAL MEASUREMENT., vol. 41, no. 6, 1 June 2020.

## III. Summary of the Invention

The invention is defined in the appended claims. One advantage of the disclosure over existing approaches to calculating a thermoregulatory strain index using core temperature is that a determination of whether the individual is coping can be detected based on a skin temperature complexity twenty (20) minutes earlier than a difference in core temperature is found, which provides an improvement in an earlier warning of the risk of HRI before current approaches. This suggests that the skin temperature complexity could be a useful tool in monitoring high intensity exercise sessions, providing useful insight into thermoregulatory control responses of the strain the thermoregulatory system is experiencing. In at least one embodiment, the heart rate complexity and skin temperature complexity are used in an TSI to identify individuals who are coping or not coping with exercise and heat strain.

Work by the inventors demonstrated that rather than absolute values or differences, time-based skin temperature complexity in conjunction with heart rate complexity was able to distinguish between two groups of people: those that appeared to be coping with heat strain and those that were not, in advance of obvious differences in core temperature, skin temperature and heart rate. In at least one embodiment, the disclosure utilizes heart rate and skin temperature signals from a physiological sensor(s) (or monitor(s)) to determine a TSI or TSI score. In at least one embodiment, the complexity of both the heart rate and skin temperature signals are used in a TSI.

One way to determine complexity is using Approximate Entropy (APEN - complexity score), alternatively other complexity approaches could be used in place of APEN. Without APEN norms or an alternative means of calculating a complexity score for skin temperature it is difficult to use the absolute numbers to conclude what is happening with the skin temperature control. A study with the participants being UK Royal Marines Commandos found that the participants fell into two groups: a HOT group that suffered from or were at risk of HRI and a WARM group that was below the HRI threshold. The difference in the scores between these groups could have two causes, either of which or both could provide insight. First, lower skin temperature APEN scores, and thus reduced complexity, in the WARM group could suggest that WARM group skin temperature is more actively controlled as a response to both the exercise and heat stress resulting in homeostatically controlled core temperature. Second, higher skin temperature APEN scores, and thus an increase in complexity, for the HOT group could suggest that skin temperature is less well controlled. This lower control could be due to the competing demands of the heat stress, exercise intensity, and maintenance of blood pressure. Thus, less active control, with less blood flow to the skin would lead to an increasing divergence in the core temperature and the skin temperature, as seen in the hot group.

The described methods and systems make use of skin temperature complexity over a predetermined measurement window as compared to heart rate complexity or alternatively with a mean heart rate over that same window. In at least one embodiment, that predetermined measurement window is adjustable, a sliding/moving measurement window, and/or 20 or 25 minutes in length. As discussed in this disclosure, TSI may take different forms including ratios, normalized, and complexity differences between rest and the current time. In at least one embodiment, the ratio of skin temperature complexity to the heart rate complexity provides a TSI as follows:

$$TSI = \frac{Tsk\_c}{HR\_c}$$

where TSI is thermoregulatory strain index, Tsk_c is skin temperature complexity, and HR_c is heart rate complexity. In this embodiment, higher values assume greater complexity and lower values assume lower complexity. In an example embodiment using APEN, TS values less than or equal to 1 indicates "coping" while TS values greater than 1 indicate increased levels of strain where the individual's thermoregulatory system is not coping with the thermal work strain it is experiencing. In an alternative embodiment, the ratio can be illustrated by

$$TSI = \frac{HR\_c}{Tsk\_c}$$

In an example embodiment using APEN, TS values greater than or equal to 1 indicates "coping" while TS values less than 1 indicate increased levels of strain where the individual's thermoregulatory system is not coping with the thermal work strain it is experiencing.

In an alternative embodiment, the TSI represents anti-rest or the inverse of the rest response. Thus, the lower heart rate complexity combine with the increased skin temperature complexity equals an increase in thermoregulatory strain as reflected by

$$TSI = 10\left(\left(ST_{comp_t} - ST_{comp_{rest}}\right) + \left(HR_{comp_{rest}} - HR_{comp_t}\right)\right)$$

where, for example, $STcomp_{rest}$ = 0.4 and is the skin temperature complexity during sleep or rest, $HRcomp_{rest}$ = 0.9 and is the HR complexity during sleep or rest, $STcomp_t$ is the current skin temperature complexity, and $HRcomp_t$ is the current heart Rate complexity. The scaling by 10 puts the index on a roughly 0 to 10 range commonly used for physiological strain index (PSI) scale. In an alternative embodiment, the complexity differences are used in the ratio embodiments discussed above.

In a further embodiment, the method includes individualizing the skin temperature and heart rate complexity scores to an individual's resting complexity values and further to normalize the scale to a simple 0 to 10 index that is the common scale for physiological strain indexes based on the work by Moran. By normalizing to the Moran PSI scale, the output is in a form that may easily be an input into other systems and/or is in a form that is commonly worked with by this field. The individualization embodiments are illustrated by:

$$TSI = 5\left(\frac{max\{(Tskc_t - Tskc_0), 0\}}{Tskc_{max} - Tskc_0}\right) + 5\left(\frac{max\{(HRc_0 - HRc_t), 0\}}{HRc_0 - HRc_{min}}\right)$$

where TSI is the thermoregulatory strain index; $Tskc_t$ is the current skin temperature complexity, $Tskc_0$ is an initial skin temperature complexity, either taken at rest or at the start of exercise (or activity); $HR_{Ct}$ is the current heart rate complexity, and $HRc_0$ is the initial heart rate complexity taken either at rest or at the beginning of exercise (or activity). In at least one embodiment, $Tskc_{max}$ is an upper limit of complexity chosen such that when an individual has this complexity their TSI score is expected to be a 10. Similarly in at least one embodiment, $HRc_{min}$ is the minimum heart rate complexity chosen such that when an individual reaches this minimum their TSI score would be expected to be 10. In at least one embodiment, the $HRc_{min}$ and the $Tskc_{min}$ are set based on appropriate bounds for a particular population, and as such is predetermined In a further embodiment, the $HRc_{min}$ and the $Tskc_{min}$ are adjusted for the individual or a safety margin.

In another method embodiment, a mean heart rate is used instead of heart ratio complexity with the ratio being compared to a logistic regression 50% decision boundary and if the comparison shows the combination of skin temperature complexity and mean heart rate to the left of the boundary corresponding to a value less than 50%, then informing the individual and/or another person that the individual is coping. If the comparison falls to the right of the boundary corresponding to a value greater than 50%, then informing the individual and/or another person that the individual is not coping. If the comparison falls along (or approximate to) the boundary, then providing an inclusive result or, alternatively, the boundary line is grouped with coping or not coping depending on the implementation.

One method includes the generation of a time series of heart rate measurements and skin temperature measurements for an individual by one or more medical sensors, e.g., a chest belt physiological monitoring system, a wrist-worn physiological monitoring system, a heart rate monitor, an electrocardiogram (ECG or EKG) monitor, a temperature sensor, a temperature thermistor, an infrared sensor, etc. During or after generation of the series of measurements for the window, determining complexity of the data series with a processor that receives the data series. In at an alternative embodiment, the processor receives the heart rate measurements and/or the skin temperature measurements for conversion in a time series of data. Determining a level of thermoregulatory strain based on the ratio of the skin temperature complexity and the heart rate complexity. The thermoregulatory strain level can be provided and/or displayed to the individual, another person, and/or an automated system to intervene when indicated by the TSI.

The TSI provides an indication of how well a particular individual is coping with their thermal work strain and thus their likelihood of succumbing to an exertional heat injury (EHI) or an HRI as compared to an overall general risk across a population or sub-grouping of that population. Early prediction of an EHI/HRI allows for earlier interventions and potentially less risk, less disruption, less long-term impact, and/or less expense associated with the EHI/HRI.

## IV. Brief Description of the Drawings

FIGs. 1A and 1B illustrate two methods according to embodiments of the invention using one or more of the described systems.
FIG. 2 illustrates a system according to at least one embodiment.
FIG. 3 illustrates a system according to at least one embodiment.
FIG. 4 illustrates a system according to at least one embodiment.
FIG. 5 illustrates a system according to at least one embodiment.
FIG. 6 illustrates a cross plot of the skin temperature (Tsk) APEN (min. 0 to 20) and mean heart rate (HR) (min. 0 to 20) HOT group (or under TS) shown as black 'X's and WARM group (or TS coping) as grey 'O's for the UK Royal Marines Commandos study, and the logistic regression 50% decision boundary is shown as a solid line.
FIG. 7 illustrates a computer program product and computer implementation according to at least one embodiment.
FIGs. 8A-9D illustrate analyzed data from a field study using in part at least one embodiment according to the invention. FIG. 8A illustrates mean heart rate and skin temperature complexities. FIG. 8B illustrates mean heart rate and mean TSI scores as determined according to at least one embodiment. FIGs. 9A-9D illustrate 4 cases of heat related illness during the field study.

## V. Detailed Description

The different embodiments share the concept and use of skir temperature complexity to gauge whether an individual is coping or not with physical exertion in their current environment. In several embodiments, the disclosure uses a heart rate complexity with the skin temperature complexity to determine a TSI, which in other embodiments, a heart rate mean is used in place of the heart rate complexity. In a further embodiment, the difference in rest complexity with the current time complexity is used to determine the TSI. In a further embodiment, the heart rate complexity and the skin temperature complexity are normalized using a resting heart rate complexity and a resting skin temperature complexity to bring the TSI into the original PSI levels and the associated levels of thermal work strain proposed by Moran et al. (1998), and PSI or a variant is widely used for determining thermal work strain.

Based on study data, using skin temperature complexity over a 20 minute or 25 minute window leads to an earlier indicator of whether the individual will cope or not cope than is possible with the core temperature-based PSIs where the core temperature is the internal core body temperature for the individual. The earlier warning allows for the individual to adjust the pace of activity, consume liquid (e.g., water) to cool down, remove a clothing layer(s), take a break, and/or make other adjustments or interventions.

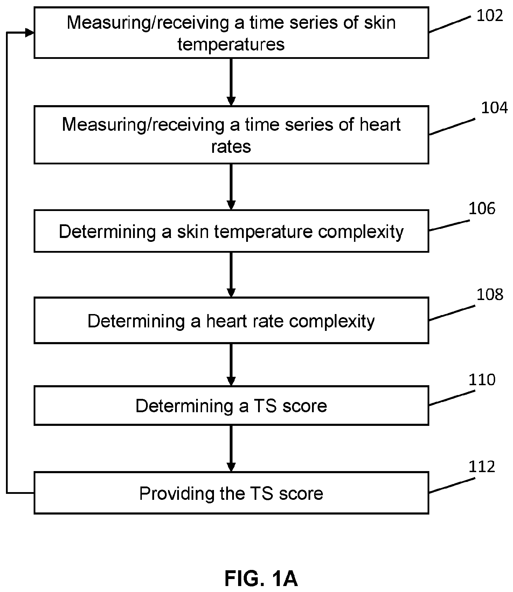

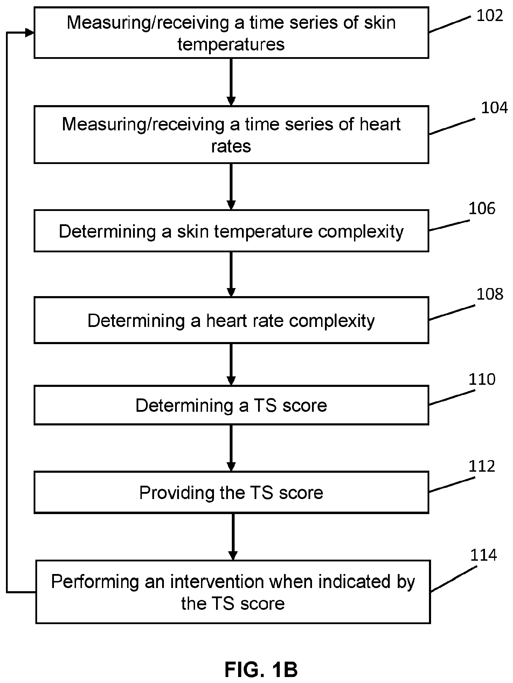

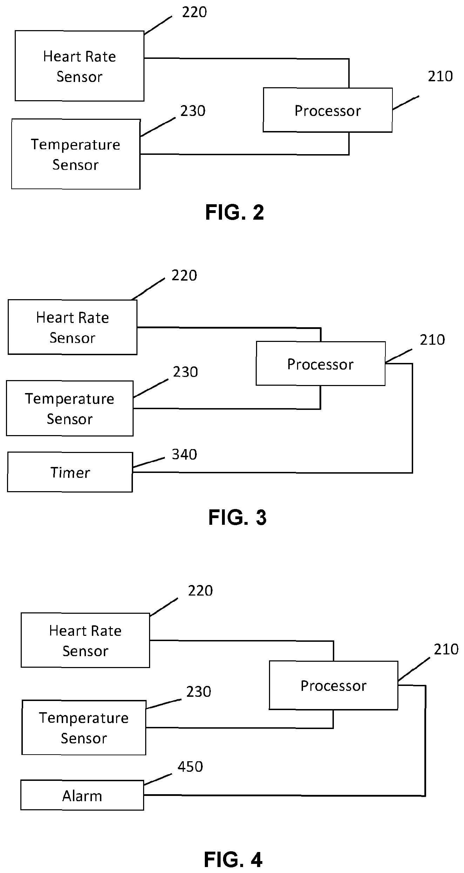

FIGs. 1A and 1B illustrate two methods for operation of at least one system embodiment like those illustrated in FIGs. 2-6. A processor 210 receives a time series of skin temperatures from at least one temperature sensor 230, 102. The processor 210 receives a time series of measured heart rates from a heart rate sensor 220, 104. In an alternative embodiment, the heart rate sensor 220 provides a continuous heart rate signal for the processor 210 to convert into a time series of heart rates. In at least one embodiment, there is one component that provides the skin temperature and the heart rate for the individual. In at least one embodiment, steps 102 and 104 can be performed in a different order and/or substantially simultaneous or substantially concurrently with each other. In most embodiments, both sensors 220, 230 will provide a signal having a continuous time series of data to the processor 210. In an alternative embodiment of the method, the time series of skin temperatures and heart rates are generated by a respective sensor 230, 220 based on their respective measurements with the time series being received by the processor 210.

The processor 210 determines a skin temperature complexity based on the skin temperature time series, 106. The processor 210 determines a heart rate complexity based on the heart rate time series, 108. The complexities may be computed using any number of complexity measures such as Approximate Entropy (APEN or ApEn), sample entropy, Shannon Entropy, Fuzzy Entropy, Multiscale Entropy, Rényi entropy, Kolmogorov complexity, entropy, or other statistical analyses for physiological data. Each of these approaches requires a time series of data points, for example 100 or more data points. In at least one embodiment, utilizing the APEN approach, a similarity time window size is set to a minimum of 2 time points but can be increased to the number of time points in the window, and the similarity input was set to 0.2 standard deviation (SD) of the measured computed across all time points, this setting can also be varied to include greater or smaller differences in the approximate entropy calculation. As an alternative, the sample entropy approach will provide better complexity numbers while likely avoiding the biases present in the APEN approach. It is preferred to use the same complexity approach for both skin temperature and heart rate to streamline the processing. In at least one embodiment, steps 106 and 108 can be performed in a different order and/or substantially simultaneous or substantially concurrently with each other.

The time series of data (i.e., skin temperatures and heart rates) is taken over a predetermined measurement window representing a time length between 5 and 30 minutes with or without the end points, between 5 and 25 minutes with or without the end points, between 10 and 25 minutes with or without the end points, between 15 and 25 minutes with or without the end points, between 17.5 and 22.5 minutes with or without the end points, between 1 and 15 minutes with or without the end points, 5 minutes, about 8 minutes, 10 minutes, 15 minutes, 20 minutes, 25 minutes, or 30 minutes. In a further embodiment, after an end of the predetermined measurement window is reached, the method will continue to determine one or both of the skin temperature and heart rate complexities using a rolling (or moving) window having the same length or shorter length of the predetermined measurement window to provide complexities on which to calculate a series of TSI scores. In a further embodiment, the complexities are measured every 30 seconds, minute, two minutes, or five minutes (although a different frequency is possible) to produce a continuous plot of the individual's TSI and/or complexities to allow for trends to be detected. In another prototype application of the method, a rolling window of 25 minutes was used with skin temperature being measured every 15 second (i.e., 100 samples) and heart rate being sampled in 5 second increments leading to 300 samples, but this allowed for the complexities to be taken over the same window to reflect the physiological response to heat and/or activity and avoids data mismatch from using different time windows. The sampling frequencies for skin temperature and heart rate could be adjust to have a similar number of measurements over the selected time window. For example, if the sampling rate for skin temperature was increased, the time window could be shortened. In an alternative embodiment, a shorter predetermined measurement window is used to start before the rolling window is lengthened with each measurement until a desired rolling window length is reached. This alternative embodiment would be advantageous as for more intense activity and/or hotter environments. Using a shorter predetermined measurement window throughout the monitoring would be advantageous when the planned activity is of a shorter duration.

The processor 210 calculates the TSI score for the individual based on the skin temperature complexity and the heart rate complexity, 110. In at least one embodiment, the TSI score is determined based exclusively on a ratio of these two complexities. In an alternative embodiment, the differences between the skin temperature complexity at rest and the time of measurement and between the heart rate complexity at rest and the time of measurement are used.

In at least one embodiment, the ratio can be determined by

$$TSI = \frac{Tsk\_c}{HR\_c} \tag{1}$$

where TSI is thermoregulatory strain index, Tsk_c is skin temperature complexity, and HR_c is heart rate complexity. In this embodiment, higher values assume greater complexity and lower values assume lower complexity. In an example embodiment using APEN, TSI scores is less than or equal to 1 indicates the individual is coping with the current thermal work strain while TSI scores is greater than 1 indicate increased levels of thermoregulatory strain where the individual's thermoregulatory system is not coping with the thermal work strain it is experiencing.

In an alternative embodiment, the ratio is flipped and is determined by

$$TS = \frac{HR\_c}{Tsk\_c} \tag{2}$$

In an example embodiment using APEN, TSI scores is greater than or equal to 1 indicates the individual is coping with the current thermal work strain while TSI scores less than 1 indicate increased levels of strain where the individual's thermoregulatory system is not coping with the thermal work strain it is experiencing.

In an alternative embodiment to either of the above illustrated ratios, the difference between the rest complexity (comp$_{rest}$) and the current complexity (comp$_t$) at time t is used instead for TSI as illustrated below

$$TSI = \frac{ST_{comp_t} - ST_{comp_{rest}}}{HR_{comp_{rest}} - HR_{comp_t}} \tag{3}$$

$$TSI = \frac{HR_{comp_{rest}} - HR_{comp_t}}{ST_{comp_t} - ST_{comp_{rest}}} \tag{4}$$

where $HR_{comprest}$ is the heart rate complexity at rest or sleeping and $ST_{comprest}$ is the skin temperature complexity at rest or sleeping.

In an alternative embodiment, the TSI score is based on the differences in each of the complexities between rest and the current time t. In at least one embodiment, the rest complexities are set based on group data instead of measured data for that individual. The rest complexity is when the individual is resting or sleeping. The relationship according to at least one embodiment is illustrated below

$$TSI = 10\left(\left(ST_{comp_t} - ST_{comp_{rest}}\right) + \left(HR_{comp_{rest}} - HR_{comp_t}\right)\right) \qquad (5)$$

where $STcomp_{rest}$ is the skin temperature complexity during sleep or rest, $HRcomp_{rest}$ is the heart rate complexity during sleep or rest, $Stcompt$ is the current skin temperature complexity at time $t$, and $HRcomp_t$ is the current heart rate complexity at time $t$. The scaling by 10 puts the index on a roughly 0 to 10 range commonly used for PSI scores and allows for use in existing systems and methods that use a PSI score. An increasing TSI represents an increasing strain on the thermoregulatory system. In an alternative embodiment, there is no scaling or different scaling is used.

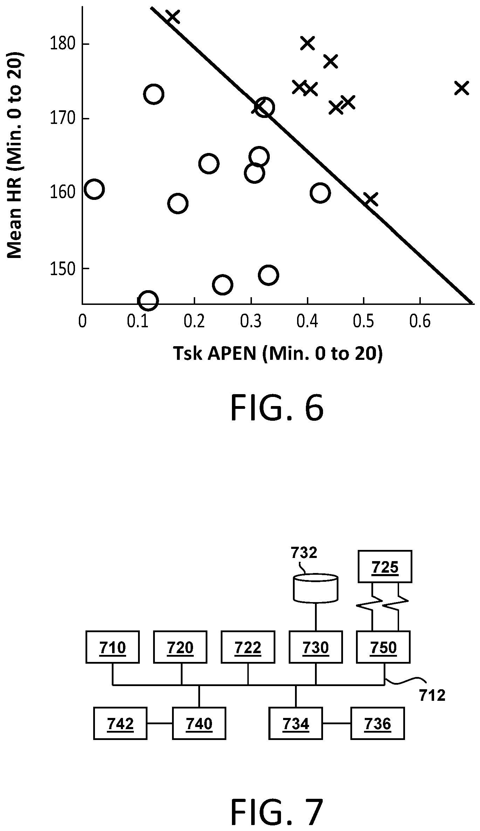

In alternative embodiment, the TSI score is determined based on the skin temperature complexity and a mean heart rate for the predetermined measurement window where the mean heart rate takes the place of the heart rate complexity, which means instead of the heart rate complexity being determined the heart rate mean is determined, 108. The ratio of the skin temperature complexity to the heart rate mean is compared to a logistic regression 50% decision boundary and if the comparison shows the combination of the skin temperature complexity and the mean heart rate to the left of the boundary, then informing the individual and/or another person that the individual is coping. If the comparison falls to the right of the boundary, then informing the individual and/or another person that the individual is not coping. If the comparison falls along the boundary, then providing an inclusive result. FIG. 6 illustrates an example of the logistic regression 50% decision boundary overlaid with the UK Royal Marines Commandos study where the "X"s represent individuals under thermoregulatory strain (TS) or potentially under TS and "O"s represent individuals under no TS.

The processor then provides the calculated TSI score, 112. The calculated TSI score may be provided to a display, a memory, a transmission system for relaying to an external device or system, and an alarm or, alternatively, producing a signal. The discussed TSI's provide an improved and earlier indication of the current physiological strain of the individual being monitored, and would allow for an activity or pace change by the individual, if possible, to lower the physiological strain before it is possible with current approaches based on the individual's core temperature. As illustrated in FIG. 1B, the individual and/or another person performs an intervention when the individual's TSI score predicts an HRI. As mentioned above, examples of an intervention include adjusting the pace of activity, administrating/drinking liquid (e.g., water) to cool down, removing a clothing layer(s), immersing the individual in lukewarm to cool water (or other fluid), applying ice packs or other cooling material, pouring lukewarm to cool water (or other fluid) over the individual, taking a break, and/or making other adjustments. A further example of an intervention would be an automated response by a system such as activation of cooling components in the clothing/equipment worn by the individual or opening of vents or similar openings in the clothing/equipment. Examples of cooling components include a cooling vest with a heat exchange system using fluid and/or activation of micro-cooling fans to move air over the individual or through the individual's equipment/clothing)

In a further embodiment, repeating the receiving (102 and 104), determining (106 and 108), calculating (110) and providing (112) steps at predetermined intervals, 114. Examples of predetermined intervals include 1 minute intervals, 5 minute intervals, 10 minute intervals, 15 minute intervals, 20 minute intervals, 30 minute intervals, 45 minute intervals, hour intervals, length of intervals between 1 minutes and 30 minutes with or without the end points, and length of intervals between 10 minutes and 2 hours with or without the end points. In a further example of the predetermined interval, the predetermined interval is equal to a fraction of the length of time of the current activity is expected to last and further such that it is proximate to any of the predetermined interval examples. In at least one embodiment, when the interval window is shortened, a similar adjustment is made to the length of the rolling window. The predetermined intervals are examples of substantially, continuously producing new TSI scores using a rolling window.

In a further embodiment, the method includes setting or selecting the predetermined interval prior to calculating the TSI score. In at least one further embodiment, a timer (or timer circuit or timing circuit) 340 illustrated in FIG. 3 can be used to delay the repeat cycle after calculating each TSI score. In at least one embodiment, the TSI score is calculated at variable times based on a change in the detected skin temperature and/or the heart rate that exceeds a predetermined threshold, the rate of a change in at least one physiological signal (e.g., skin temperature or heart rate) over a predetermined change time, the rate of a change in the skin temperature complexity, the rate of a change in the heart rate complexity, variances in any of these, or a combination of these. In at least one embodiment, the timer 340 is used to measure the complexity window length and in a further embodiment the time 340 provides a signal to the processor 210. In at least one embodiment, the predetermined intervals for calculating the TSI scores are supplemented by calculations at variable times.

In at least one further embodiment, when the TSI score exceeds a predetermined alarm threshold (e.g., a TSI score that indicates action is needed), an alert is generated by an alarm 450 of FIG. 4. In at least one embodiment, the processor 210 provides an alarm signal to the alarm 450 that triggers the alert. In an alternative embodiment, when a rate of change of TSI or one of the complexities exceeds a predetermined rate alarm threshold, an alert is generated.

In a further embodiment, the method includes individualizing the skin temperature and heart rate complexity scores to an individual's resting values and further to normalize the scale to a simple 0 to 10 index that is the common scale for PSIs based on previous heat strain scales such as the Moran PSI. By normalizing to the Moran PSI scale, the output is in a form that may easily be an input into other systems and/or is in a form that is commonly worked with by this field. Table 1 shows the original PSI levels and the associated levels of thermal work strain according to Moran et al. (1998):

Table 1

| PSI | Strain |
|-----|--------|
| 0 | |
| 1 | No/Little |
| 2 | |
| 3 | Low |
| 4 | |
| 5 | Moderate |
| 6 | |
| 7 | High |
| 8 | |
| 9 | Very High |
| 10 | |

The individualization embodiments may use the following relationship to normalize the thermoregulatory strain to the PSI:

$$TSI = 5\left(\frac{max\{(Tskc_t - Tskc_0),0\}}{Tskc_{max} - Tskc_0}\right) + 5\left(\frac{max\{(HRc_0 - HRc_t),0\}}{HRc_0 - HRc_{min}}\right) \qquad (6)$$

where TSI is the thermoregulatory strain index; $Tskc_t$ is the current skin temperature complexity, $Tskc_0$ is an initial skin temperature complexity, either taken at rest or at the start of exercise; $HR_{ct}$ is the current heart rate complexity, and $HRc_0$ is the initial heart rate complexity taken either at rest or at the beginning of exercise. In at least one embodiment, $Tskc_{max}$ is an upper limit of complexity chosen such that when an individual has this complexity their TSI score is expected to be a 10. Similarly in at least one embodiment, $HRc_{min}$ is the minimum heart rate complexity chosen such that when an individual reaches this minimum their TSI score would be expected to be 10. In at least one embodiment when the difference of complexities for the skin temperature and/or the heart rate is negative, then that term becomes zero as illustrated in the equation above. In at least one embodiment, the $HRc_{min}$ and the $Tskc_{max}$ are set based on appropriate bounds for a particular population, and as such is predetermined. Using study data, like that discussed *infra,* these complexities can be set using, for example, the 95% or 97.5% measurements. It is envisioned that these numbers could be adjusted in a manner similar to estimating maximum heart rate for a person based on age (i.e., 220-age). In a further embodiment, the $HRc_{min}$ and the $Tskc_{min}$ are adjusted for the individual based on previous testing to determine these complexities or a safety margin.

In at least one embodiment as illustrated, for example in FIG. 2, the methods discussed in connection with FIGs. 1A and 1B are performed on the processor 210 running code that enables the performance of at least one method embodiment and is in communication with the heart rate sensor 220 and the temperature sensor 230. In an alternative embodiment, one or both sensors are external to the system. In at least one embodiment, the communication is electrical communication, which includes a wired connection and wireless signal communications other than an optical connection. Examples of a heart rate sensor 220 include a heart rate monitor attached to the individual, a chest belt physiological monitoring system, a wrist-worn physiologically monitoring system, an electrocardiogram (ECG or EKG) monitor, a processor for receiving ECG or EKG signals from electrodes attached to the person, a processor for receiving a photoplethysmogram signal (e.g., a pulse oximeter), a pulse oximeter, or a processor for receiving a ballistic-cardiogram signal. The processor used as part of the heart rate sensor 220 in at least one embodiment is the processor 210. Examples of a temperature sensor 230 configured to detect a temperature on the skin of the individual being monitored, such as an expanse of skin, can include various analog and digital temperature sensors, a temperature thermistor, and infrared thermometers. As is known by a person having ordinary skill in the art, temperature sensors are impacted by the environmental temperature and the level of direct sunlight. One solution is to shield the temperature sensor from direct sunlight and/or environmental conditions by the presence of a physical enclosure or clothing layers over the temperature sensor, and as discussed *infra* participants in a field study wore an infrared temperature sensor in a puck enclosure against their chest while wearing a lightweight t-shirt and fatigues. In a further embodiment, the temperature sensor 230 is shielded. In at least one embodiment, there is a memory, data storage, or storage (not illustrated) in communication with the processor 210. In at least one embodiment, the storage stores resting complexities for the skin temperature and the heart rate, the maximum skin temperature complexity, and/or the minimum heart rate complexity.

In at least one embodiment of the previous embodiments, for example as illustrated in FIG. 3, there is an optional component of the timer (or timer circuit) 340 for setting or scheduling the determination times (or intervals) or measurement windows at which the heart rate complexity and the skin temperature complexity are used. In an alternative embodiment, the predetermined intervals are set by a user or the individual being monitored. In a further alternative embodiment, the predetermined time period is stored in a memory or data storage, for example, on a memory chip located on the individual's wrist, in a database located on a network, or is present in the code running on the processor 210. The timer 340, for example, can adjust the determination times for the measurement windows. The timer 340 can transmit a signal to the processor 210 notifying the processor 210 to perform at least one instruction, such as alerting the processor 210 that a time interval has occurred. The timer 340 can be an integrated circuit, chip, or microchip used for timing, pulse, and/or oscillator applications.

In at least one further embodiment to any of the embodiments as illustrated in FIG. 4, the system includes the alarm 450 or another similar component to produce an alert indicating the person being monitored has exceeded an alarm parameter threshold for the TSI score as calculated by the processor 210. The alarm 450 can be contained within the system or in communication with the system to produce an alert. The alert can be produced in any sensory form, such as auditory output through a speaker, visual output through a display and/or light elements, and/or a vibration from a transducer, configured to alert the individual or a monitoring system that the alarm parameter threshold has been exceeded.

In at least one embodiment, the processor 210, the heart rate sensor 220, the temperature sensor 230, and/or the other described electronics, such as the timer 340, or the alarm 450 embodied in the block diagrams of FIGs. 2-4, are housed within or attached to an apparatus worn by an individual being monitored, such as on the individual's chest, arm, or wrist, but is not limited in this regard.

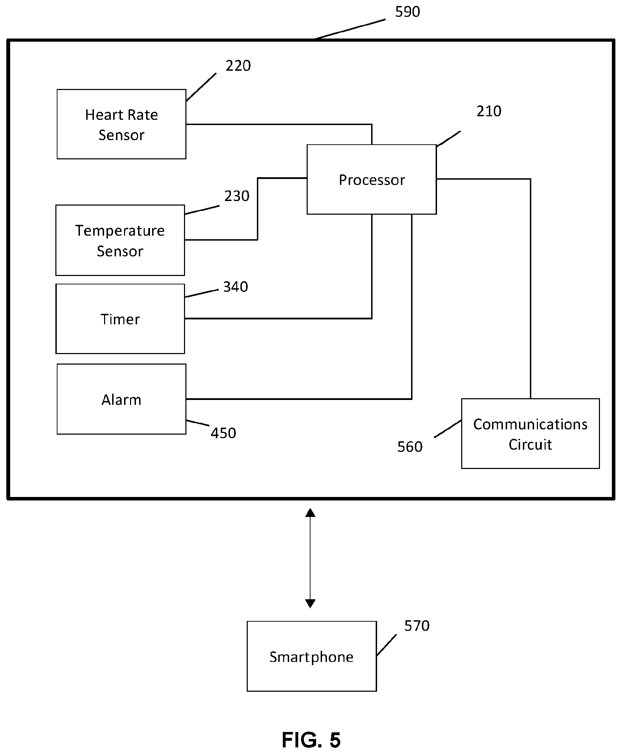

In a further embodiment illustrated in FIG. 5, the processor 210, the timer 340, and the alarm 450 are present in one housing 590, such as that provided by a smartphone or a smartwatch, in electrical communication with the heart rate sensor 220 and/or the temperature sensor 230. In a further embodiment, the heart rate sensor 220 and/or the temperature sensor 230 are part of the smartwatch or other body worn housing. In a further embodiment or in addition to the previous embodiments, the skin temperature, the heart rate, and/or the TSI score can be shown on a display present on the wearable device, such as a wrist worn display, a smartphone, or a heads-up display, for viewing by the person being monitored.

In at least one embodiment, the processor 210 is detached from the individual being monitored and is located in external equipment such as a medical monitor or a computer implemented device running code according to at least one method embodiment. In such an embodiment, examples of how the information is sent to such external equipment include, but is not limited to, transmitting can be sent wirelessly including optically, or by various types or arrangements of hardwire connections, or combinations thereof. An example of wireless and optical transmissions is through a transmitter and a receiver. In a further embodiment to any of the previous embodiments, the information can be received through, for example, a user interface, such as a keyboard, graphical user interface (e.g., touchscreen) on a display, or a microphone.

The information and operations that are transmitted throughout the various described embodiments can be in the form of electronic data, wireless signals, or a variation thereof, for example. In at least one embodiment, the processor 210 can be designed to accomplish signal processing in the configured system containing the sensors and electronics but can transmit signals to a network for further processing. In another embodiment, the processor 210 is connected to a communications circuit 560 to transmit individual or time series skin temperatures, individual or time series heart rates, and/or the TS score to an external system for monitoring and/or display, for example to the Individual Heat Optimization Training Tool (iHOTT) system that uses a color code system for monitored individuals to indicate if they are good (green), check on them (yellow), check on them now (red), and heat stroke alert (black) where the thermoregulatory strain index could be used to categorize the monitored individuals into these categories. FIG. 5 illustrates the communications circuit 560 configured to communicate directly with the external system, such as the communication circuit 560 communicating directly with a smartphone 570, a body worn computer, and/or a tablet with the individual and/or another person or a stand-alone computing device. The information and operations that are transmitted throughout the various embodiments can be sent wirelessly, optically, or by various types or arrangements of hard wire connections, or combinations thereof, among the various system components, for example.

In a further embodiment, the system includes one or more means instead of a particular component.

A heart rate means for detecting a heart rate includes a sensor for measuring heart beats or blood flow, a heart rate sensor, a heart monitor, or another biotelemetry device configured to detect a heartbeat, a heart rate, or blood flow but is not limited in this regard and the means for measuring a heart rate or a heartbeat can be measured in real time or recorded for later use. The heart rate means includes the previous examples of the heart rate sensor.

In at least one embodiment, a temperature means for measuring the skin temperature can include various manual or digital thermometer and temperature gauges, but is not limited in this regard and additional apparatuses configured to detect heat or temperature can be used. The temperature means can detect skin temperature of an area of a body, such as an area of skin, can include a manual or digital thermometer, a temperature gauge, for example but is not limited in this regard and additional apparatuses configured to detect heat or temperature of an area of a body can be used. The temperature means includes the previous examples of the temperature sensor.

In at least one embodiment, a calculation means for calculating a TSI score for the individual based on the detected skin temperatures and the detected heart rates is the processor with suitable programming to perform the steps associated with this function.

As will be appreciated by one skilled in the art based on this disclosure, aspects of the disclosure may be embodied as a system, method or computer program product. Accordingly, aspects of the disclosure may take the form of an entirely hardware embodiment, a processor operating with software embodiment (including firmware, resident software, micro-code, etc.) or an embodiment combining software and hardware aspects that may all generally be referred to herein as a "circuit," "module" or "system." Furthermore, aspects of the disclosure may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable (or USB) drive, a hard drive, a random-access memory (RAM), a read-only memory (ROM), an erasable programmable read-only memory (EPROM or Flash memory), a magnetic storage device, or any suitable combination of the foregoing. In the context of this disclosure, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Computer program code for carrying out operations for aspects of the disclosure may be written in any combination of one or more programming languages. The program code may execute entirely on the processor 210 associated with the individual (individual's processor), partly on the individuals' processor, as a stand-alone software package, partly on the individual's processor and partly on a remote computer or entirely on the remote computer or server. In the latter scenario, the remote computer may be connected to the individual's processor through any type of network, including Bluetooth, a local area network (LAN) or a wide area network (WAN), or the connection may be made to an external computer.

Aspects of the disclosure are described above with reference to flowchart illustrations and/or block diagrams of methods, systems (or apparatuses) and computer program products according to embodiments. It will be understood that each block of the flowchart illustrations and/or block diagrams, and combinations of blocks in the flowchart illustrations and/or block diagrams, can be implemented by computer program instructions. These computer program instructions may be provided to the individual's processor, the processor of a general-purpose computer, a special purpose computer, or other programmable data processing apparatus to produce a machine, such that the instructions, which execute with the processor of the computer or other programmable data processing apparatus, create means for implementing the functions/acts specified in the flowchart and/or block diagram block or blocks.

These computer program instructions may also be stored in a computer readable medium that can direct the individual's processor, a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto the individual's processor, a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

Referring now to FIG. 7, a representative hardware environment for practicing at least one embodiment of the disclosure is depicted. This schematic drawing illustrates a hardware configuration of an information handling/computer system in accordance with at least one embodiment. The system includes at least one processor or central processing unit(s) (CPU) 710. The CPU(s) 710 are interconnected with system bus 712 to various devices such as a random-access memory (RAM) 720, read-only memory (ROM) 722, and an input/output (I/O) adapter 730. The I/O adapter 730 can connect to peripheral devices such as disk units 732 or other program storage devices that are readable by the system. The system can read the inventive instructions on the program storage devices and follow these instructions to execute the methodology of at least one embodiment. The system further includes a user interface adapter 740 that connects a user interface device such as a touch screen device 742 to the bus 712 to gather user input. Additionally, a communication adapter 750 connects the bus 712 to a data processing network 752, and a display adapter 734 connects the bus 712 to a display device 736 which may be embodied as an output device such as a monitor or transmitter, for example. In at least one embodiment, the touch screen device 742 and the display device 736 are the same component.

Field Study Data

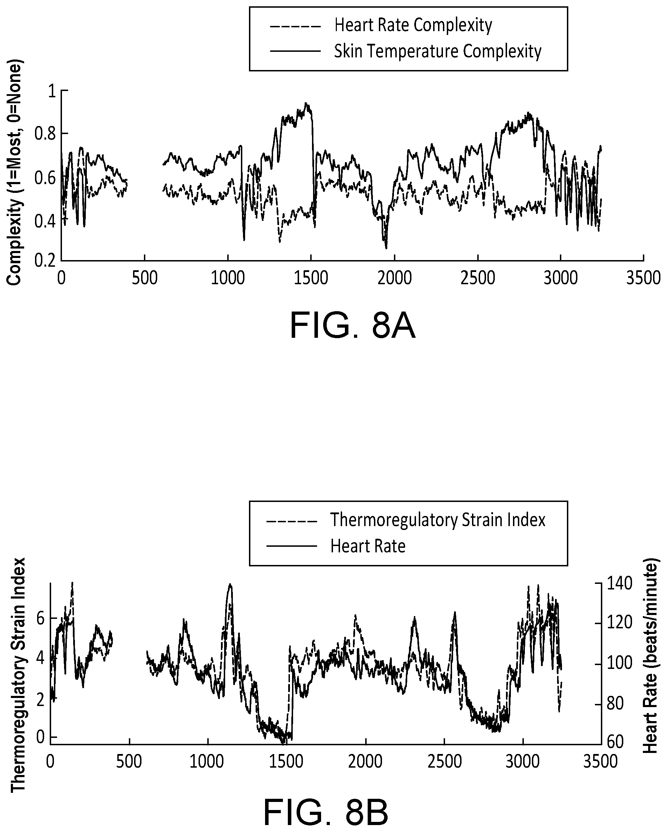

During the summer of 2021, a series of field research studies were done to test a prototype system and the feasibility to monitor about 500 recruits during a 56 hour final field training exercise (i.e., crucible). As part of the field study baseline physiological and perceptual data to characterized the thermoregulatory stresses of the crucible and capture data of exertional heat illnesses as they occurred. During the crucibles both heart rate and skin temperature were collected from each of the participants using a monitoring device. Skin temperature and heart rate complexities were computed using APEN for 361 recruits who participated in the first monitored crucible in 2021. The parameters used for APEN were 0.2 SD of individuals, comparing 2 points, and about 100 points int eh time interval. FIG. 8A illustrates the mean heart rate complexity and the mean skin temperature complexity for the duration of the crucible. FIG. 8B illustrates the mean heart rate and the mean TSI (or series of TSI scores) using equation 5 above with the skin temperature complexity at rest being set to 0.4 and the heart rate complexity at rest being set to 0.9.

FIG. 8A illustrates heart rate and skin temperature complexities during the crucible. The general crucible periods from FIG. 8B can be seen in the complexity data albeit the heart rate and the skin temperature respond different to periods of rest and movement. Overall, the heart rate complexity is as expected from the literature where the higher heart rate complexities are during sleep/rest to more regular during periods of activity while skin temperature complexity responds differently with its most regular periods being during sleep to more complexity during periods of activity.

FIG. 8B illustrates six periods of interest that illustrate different activities during the crucible. Periods of interest are noted on the chart with the numbers 1-6. Period 1 is at the start of crucible (Thursday 2:00am) road march to the field location with two periods of movement with a rest in between where the maximum mean heart rate was about 120 beats per minute (bpm). Period 2 is during a day night movement where the peak mean heart rate was about 140 bpm. Period 3 was the first night's sleep. Period 4 was a night movement during the second day where the peak mean heart rate was about 130 bpm. Period 5 was the second night's sleep. Period 6 was at the crucible completion. The final extended night was a return back to their barracks. FIG. 8B illustrates 4 periods of movement with 3 breaks. The mean heart rate increases from the first movement peak of about 110 bpm to the last movement peak of about 130 bpm.

The TSI is represented by anti-rest or the inverse of the rest response where a lower heart rate complexity combined with an increased skin temperature complexity results in a higher thermoregulatory strain (equation 5):

$$TSI = 10\left(\left(ST_{comp_t} - ST_{comp_{rest}}\right) + \left(HR_{comp_{rest}} - HR_{comp_t}\right)\right) \qquad (5)$$

where $STcomp_{rest}$ = 0.4 and $HRcomp_{rest}$ = 0.9

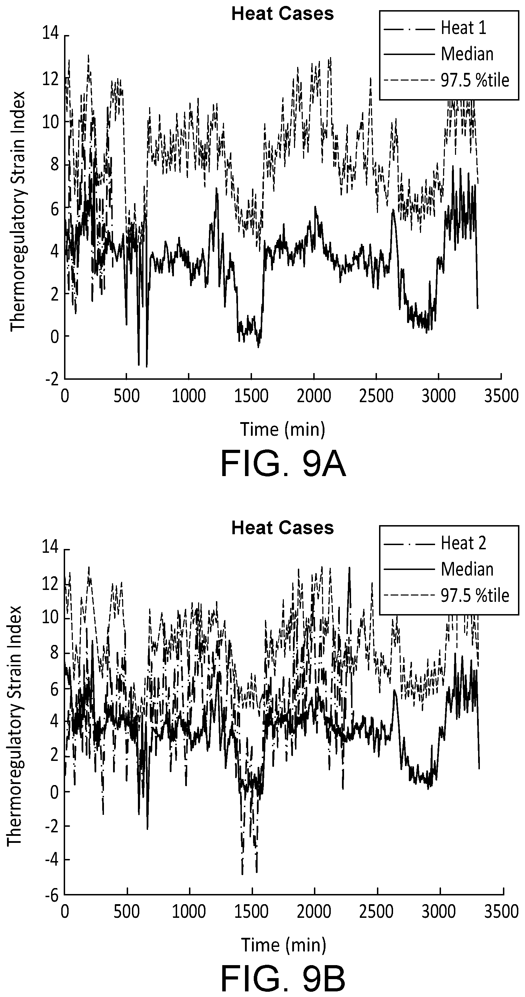

FIGs. 9A-9D illustrate the TSI for four heat cases as compared to the group mean TSI score and a 97.5% tile (dashed line). The 97.5% tile represents individuals who experienced heat illness had TSI scores that were at the extreme high end compared to 97.5% of all other participants. The mean TSI tracks the heart rate well showing increased stress during periods of movement and low stress at night. Interestingly, the TSI is not 100% correlated with the heart rate. The TSI shows a greater strain during period 1 compared to period 2, while the heart rate is greater in period 2. Also, while the peak heart rates for the two movements in period 1 are similar, the TSI increases. Conversely, in period 6, the heart rate peaks increase for subsequent movements while the TSI decreases.

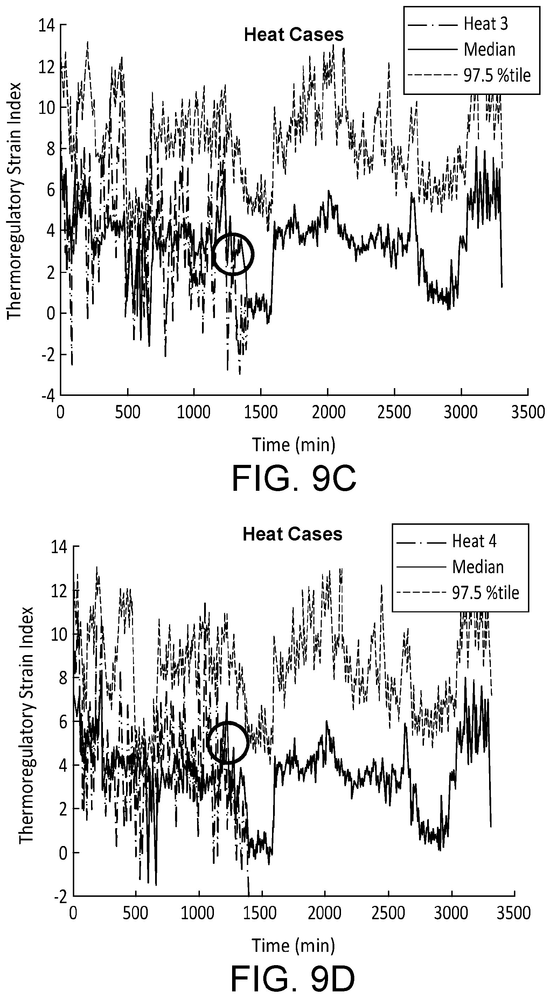

Applying TSI to the 4 individuals that experienced an exertional heat illness shows a similar pattern from the data. FIGs. 9A-9D illustrate the mean TSI of all of the crucible participants along with the 97.5 percentile from the data. All of the heat cases show high TSI values with many of those values close to or exceeding the 97.5% of TSI for all crucible participants during the same periods of activity prior to being medically removed from the training event, which is reflected by the end of their TSI line. FIG. 9A illustrates how the first case would have been identified well before the first case rose to medical treatment around 1300 hours (11 hours into the training). There was no corresponding time for the second case illustrated in FIG. 9B, but given the number of high values over the approximately 41 hours that medical care would be required. FIG. 9C illustrates how the third case would have been identified well before it rose to medical treatment around 22:30 hours (21.5 hours into the training). FIG. 9D illustrates how the fourth case would have been identified well before it rose to medical treatment around 22:00 hours (21 hours into the training). FIGs. 9C and 9D use a circle to identify the point the heat illness was diagnosed.

Currently, an estimated core temperature approach would provide an alert on two of these cases close to the time of collapse. The TSI has the potential to alert trainers and medics earlier that an individual is struggling and should be more closely watch/assessed.

The flowchart and block diagrams in the figures illustrate the architecture, functionality, and operation of possible implementations of systems, methods and computer program products according to various embodiments of the disclosure. In this regard, each block in the flowchart or block diagrams may represent a module, circuit, segment, or portion of code, which includes one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the root terms "include" and/or "have", when used in this specification, specify the presence of stated features, integers, steps, operations, elements, and/or components, but do not preclude the presence or addition of one or more other features, integers, steps, operations, elements, components, and/or groups thereof. As used, "A, B and/or C" or "at least one of A, B, and C" means just A; just B; just C; A and B; A and C; B and C; or A, Band C.

The corresponding structures, materials, acts, and equivalents of all means plus function elements in the claims below are intended to include any structure, or material, for performing the function in combination with other claimed elements as specifically claimed. The description of the disclosure has been presented for purposes of illustration and description, but is not intended to be exhaustive or limited.

Although the disclosure has been described in terms of particular example embodiments, it is not limited to those embodiments. The embodiments, examples, and modifications which would still be encompassed by the invention may be made by those skilled in the art, particularly in light of the foregoing teachings.

As used above "substantially," "generally," and other words of degree are relative modifiers intended to indicate permissible variation from the characteristic so modified. It is not intended to be limited to the absolute value or characteristic which it modifies but rather possessing more of the physical or functional characteristic than its opposite, and preferably, approaching or approximating such a physical or functional characteristic.

Those skilled in the art will appreciate that various adaptations and modifications of the embodiments described above can be configured without departing from the scope of the invention as defined in the appended claims.

### VI. Industrial Applicability

The disclosed embodiments provide an improved way to detect an EHI/HRI based upon an individual's TSI or a particular TSI score based on the skin temperature complexity and the heart rate complexity. In some embodiments, the systems and methods provide a mechanism to alert the individual of the potential for an EHI/HRI based on their TSI. The detection and prediction allow for an intervention to occur for the individual.

### Claims

1. A method for generating a thermoregulatory strain index (TSI) for an individual, the method comprising:

generating a time series of heart rates with at least one heart rate sensor configured to be attached to or placed on the individual;
generating a time series of skin temperatures with at least one temperature sensor configured to detect a skin temperature of the individual;
receiving the time series of heart rates and the time series of skin temperatures with a processor in electrical communication with the at least one temperature sensor and the at least one heart rate sensor;

determining a heart rate complexity by the processor from the heart rate time series;
determining a skin temperature complexity by the processor from the skin temperature time series;
calculating a TSI score by the processor based on a ratio of the skin temperature complexity and the heart rate complexity, wherein the ratio is:

$$TSI = \frac{Tsk\_c}{HR\_c}$$

where TSI is the thermoregulatory strain index score, Tsk_c is the skin temperature complexity, and HR_c is the heart rate complexity, wherein the complexities are determined using Approximate Entropy, and
any TSI score equal to or less than 1 indicates the individual is coping with a current thermal work strain while any TSI score greater than 1 indicates the individual is not coping with the current thermal work strain;
producing an output signal for the TSI score, optionally displaying the TSI score on a display based on the output signal; and
optionally performing an intervention for the individual by the individual, another person, and/or an automated system, and
wherein the heart rate time series and the skin temperature time series are over identical measurement windows.

2. The method according to claim 1, further comprising

retrieving from a data storage a resting heart rate complexity and a resting skin temperature complexity of the individual; and
normalizing the heart rate complexity with the resting heart rate complexity and normalizing the skin temperature complexity with the resting skin temperature complexity to produce the TSI score on a scale of 1 to 10.

3. The method according to claim 1 or claim 2, further comprising calculating a new calculated TSI score at intervals based on one or more variances in the measured skin temperature, the measured heart rate, the skin temperature complexity, and/or the heart rate complexity exceeding a threshold in at least one of the skin temperatures and the heart rates received by the processor.

4. The method according to any one of claims 1-3, further comprising calculating a new calculated TSI score based on calculating at least one first TSI score at an initial time designation from a timer and calculating one new calculated TSI score at each predetermined time interval based on a signal from a timer circuit.

5. The method according to any one of claims 1-4, further comprising substantially, continuously calculating new calculated TSI scores using a rolling window for the heart rate time series and the skin temperature time series, and optionally the rolling window has a length of 20 minutes or 25 minutes.

6. The method according to any one of claims 1-5, further comprising generating an alert signal from the processor to an alarm when the TSI score exceeds a predetermined alarm threshold.

7. A system for generating a thermoregulatory strain index (TSI) for an individual, the system comprising:

a processor configured to

receive a time series of temperature readings from a temperature sensor attached to a skin of the individual,
receive a time series of heart rates or a heart rate signal from a heart rate sensor attached to the individual, and
produce a TSI score for the individual using the time series of heart rates or the heart rate signal from the heart rate sensor and the time series of skin temperatures from the temperature sensor by

determining a heart rate complexity or a mean heart rate from the heart rate time series or the heart rate signal,
determining a skin temperature complexity from the skin temperature time series,

calculating a TSI score based on a ratio of the skin temperature complexity and the heart rate complexity, wherein the ratio is:

$$TSI = \frac{Tsk\_c}{HR\_c}$$

where TSI is the thermoregulatory strain index score, Tsk_c is the skin temperature complexity, and HR_c is the heart rate complexity, wherein the complexities are determined using Approximate Entropy, and any TSI score equal to or less than 1 indicates the individual is coping with a current thermal work strain while any TSI score greater than 1 indicates the individual is not coping with the current thermal work strain, and

producing an output signal for the TSI score;

an optional display in electrical communication with said processor and configured to display the TSI score from the output signal received from said processor; and
an optional communications module configured to communicate the output signal with an external device.

**Patentansprüche**

1.  Verfahren zum Erzeugen eines thermoregulatorischen Belastungsindex (thermoregulatory strain index, TSI) für eine Person, wobei das Verfahren umfasst:

    Erzeugen einer Herzfrequenz-Zeitreihe mit mindestens einem Herzfrequenzsensor, der konfiguriert ist, um an der Person angebracht oder auf dieser platziert zu werden;
    Erzeugen einer Hauttemperatur-Zeitreihe mit mindestens einem Temperatursensor, der konfiguriert ist, um eine Hauttemperatur der Person zu erfassen;
    Empfangen der Herzfrequenz-Zeitreihe und der Hauttemperatur-Zeitreihe mit einem Prozessor, der in elektrischer Verbindung mit dem mindestens einen Temperatursensor und dem mindestens einen Herzfrequenzsensor steht;
    Bestimmen einer Herzfrequenzkomplexität durch den Prozessor aus der Herzfrequenz-Zeitreihe;
    Bestimmen einer Hauttemperaturkomplexität durch den Prozessor aus der Hauttemperatur-Zeitreihe;
    Berechnen eines TSI-Wertes durch den Prozessor auf der Grundlage eines Verhältnisses der Hauttemperaturkomplexität und der Herzfrequenzkomplexität, wobei das Verhältnis

$$TSI = \frac{Tsk\_c}{HR\_c}$$

    ist und TSI der Wert des thermoregulatorischen Belastungsindex, Tsk_c die Komplexität der Hauttemperatur und HR_c die Komplexität der Herzfrequenz ist, wobei die Komplexitäten unter Verwendung der approximativen Entropie bestimmt werden, und
    jeder TSI-Wert, der gleich oder kleiner als 1 ist, anzeigt, dass die Person mit einer aktuellen thermischen Arbeitsbelastung zurechtkommt, während jeder TSI-Wert, der größer als 1 ist, anzeigt, dass die Person mit der aktuellen thermischen Arbeitsbelastung nicht zurechtkommt;
    Erzeugen eines Ausgangssignals für den TSI-Wert, optional Anzeigen des TSI-Werts auf einem Anzeigegerät auf der Grundlage des Ausgangssignals; und
    optional Durchführen einer Intervention für die Person durch die Person selbst, eine andere Person und/oder ein automatisiertes System, und
    wobei die Herzfrequenz-Zeitreihe und die Hauttemperatur-Zeitreihe über identische Messfenster verlaufen.

2.  Verfahren nach Anspruch 1, das ferner umfasst

    Abrufen einer Ruheherzfrequenzkomplexität und einer Ruhehauttemperaturkomplexität der Person aus einem Datenspeicher; und
    Normieren der Herzfrequenzkomplexität mit der Ruheherzfrequenzkomplexität und Normieren der Hauttemperaturkomplexität mit der Ruhehauttemperaturkomplexität, um den TSI-Wert auf einer Skala von 1 bis 10 zu erzeugen.

3.  Verfahren nach Anspruch 1 oder Anspruch 2, das ferner umfasst: Berechnen eines neuen berechneten TSI-Wertes in Intervallen auf der Grundlage einer oder mehrerer Abweichungen der gemessenen Hauttemperatur, der gemessenen Herzfrequenz, der Hauttemperaturkomplexität und/oder der Herzfrequenzkomplexität, die einen Schwellenwert der vom Prozessor empfangenen Hauttemperaturen und/oder Herzfrequenzen überschreiten.

4. Verfahren nach einem der Ansprüche 1 bis 3, das ferner umfasst: Berechnen eines neuen berechneten TSI-Wertes, basierend auf dem Berechnen mindestens eines ersten TSI-Wertes zu einem von einem Zeitgeber festgelegten Anfangszeitpunkt und dem Berechnen eines neuen berechneten TSI-Wertes in jedem vorgegebenen Zeitintervall basierend auf einem Signal von einer Zeitgeberschaltung.

5. Verfahren nach einem der Ansprüche 1 bis 4, das ferner umfasst: im Wesentlichen kontinuierliches Berechnen von neuen berechneten TSI-Werten unter Verwendung eines gleitenden Fensters für die Herzfrequenz-Zeitreihen und die Hauttemperatur-Zeitreihen, wobei optional das gleitende Fenster eine Länge von 20 Minuten oder 25 Minuten hat.

6. Verfahren nach einem der Ansprüche 1 bis 5, das ferner umfasst: Ausgeben eines Warnsignals vom Prozessor an eine Warnvorrichtung, wenn der TSI-Wert einen vorbestimmten Warnschwellenwert überschreitet.

7. System zum Erzeugen eines thermoregulatorischen Belastungsindex (TSI) für eine Person, wobei das System umfasst:

einen Prozessor, der konfiguriert ist zum

Empfangen einer Temperaturmesswert-Zeitreihe von einem an der Haut der Person angebrachten Temperatursensor,
Empfangen einer Herzfrequenz-Zeitreihe oder eines Herzfrequenzsignals von einem an der Person angebrachten Herzfrequenzsensor und
Erzeugen eines TSI-Wertes für die Person unter Verwendung der Herzfrequenz-Zeitreihe oder des Herzfrequenzsignals vom Herzfrequenzsensor und der Hauttemperatur-Zeitreihe vom Temperatursensor durch Bestimmen einer Herzfrequenzkomplexität oder einer mittleren Herzfrequenz aus der Herzfrequenz-Zeitreihe oder dem Herzfrequenzsignal,
Bestimmen einer Hauttemperaturkomplexität aus der Hauttemperatur-Zeitreihe,
Berechnen eines TSI-Wertes auf der Grundlage eines Verhältnisses der Hauttemperaturkomplexität und der Herzfrequenzkomplexität, wobei das Verhältnis

$$TSI = \frac{Tsk\_c}{HR\_c}$$

ist und TSI der Wert des thermoregulatorischen Belastungsindex ist, Tsk_c die Hauttemperaturkomplexität und HR_c die Herzfrequenzkomplexität ist, wobei die Komplexitäten unter Verwendung der approximativen Entropie bestimmt werden, und jeder TSI-Wert, der gleich oder kleiner als 1 ist, anzeigt, dass die Person mit einer aktuellen thermischen Arbeitsbelastung zurechtkommt, während jeder TSI-Wert, der größer als 1 ist, anzeigt, dass die Person mit der aktuellen thermischen Arbeitsbelastung nicht zurechtkommt, und Erzeugen eines Ausgangssignals für den TSI-Wert;

optional eine Anzeigevorrichtung, die in elektrischer Verbindung mit dem Prozessor steht und konfiguriert ist, um den TSI-Wert aus dem vom Prozessor empfangenen Ausgangssignal anzuzeigen; und
optional ein Kommunikationsmodul, das konfiguriert ist, um das Ausgangssignal an eine externe Vorrichtung zu übertragen.

**Revendications**

1. Procédé pour générer un indice de contrainte thermorégulatoire (TSI) pour un individu, le procédé comprenant :

générer une série chronologique de fréquences cardiaques à l'aide d'au moins un capteur de fréquence cardiaque configuré pour être fixé ou placé sur l'individu ;
générer une série chronologique de températures cutanées à l'aide d'au moins un capteur de température configuré pour détecter la température cutanée de l'individu ;
recevoir la série chronologique de fréquences cardiaques et de la série chronologique de températures cutanées à l'aide d'un processeur en communication électrique avec au moins un capteur de température et au moins un capteur de fréquence cardiaque ;

déterminer une complexité de fréquence cardiaque par le processeur à partir de la série chronologique de fréquences cardiaques ;

déterminer une complexité de la température cutanée par le processeur à partir de la série chronologique de températures cutanées ;

calculer un score TSI par le processeur sur la base d'un rapport entre la complexité de la température cutanée et la complexité de la fréquence cardiaque, où le rapport est :

$$TSI = \frac{Tsk\_c}{HR\_c}$$

où TSI est le score de l'indice de contrainte thermorégulatrice, Tsk_c est la complexité de la température cutanée et HR_c est la complexité de la fréquence cardiaque, les complexités étant déterminées à l'aide de l'entropie approximative, et

tout score TSI égal ou inférieur à 1 indique que l'individu fait face à une contrainte thermique, tandis que tout score TSI supérieur à 1 indique que l'individu ne fait pas face à la contrainte thermique;

produire un signal de sortie pour le score TSI, afficher éventuellement le score TSI sur un écran en fonction du signal de sortie ; et

effectuer éventuellement une intervention pour l'individu par l'individu, une autre personne et/ou un système automatisé, et

dans lequel la série chronologique de fréquence cardiaque et la série chronologique de température cutanée sont sur des fenêtres de mesure identiques.

2. Procédé selon la revendication 1, comprenant en outre

la récupération, à partir d'un stockage de données, d'une complexité de la fréquence cardiaque au repos et d'une complexité de la température cutanée au repos de l'individu ; et

la normalisation de la complexité de la fréquence cardiaque avec la complexité de la fréquence cardiaque au repos et la normalisation de la complexité de la température cutanée avec la complexité de la température cutanée au repos afin de produire le score TSI sur une échelle de 1 à 10.

3. Procédé selon la revendication 1 ou la revendication 2, comprenant en outre le calcul d'un nouveau score TSI calculé à intervalles réguliers sur la base d'une ou plusieurs variations de la température cutanée mesurée, de la fréquence cardiaque mesurée, de la complexité de la température cutanée et/ou de la complexité de la fréquence cardiaque dépassant un seuil dans au moins l'une des températures cutanées et des fréquences cardiaques reçues par le processeur.

4. Procédé selon l'une quelconque des revendications 1 à 3, comprenant en outre le calcul d'un nouveau score TSI calculé sur la base du calcul d'au moins un premier score TSI à un moment initial désigné par une minuterie et du calcul d'un nouveau score TSI calculé à chaque intervalle de temps prédéterminé sur la base d'un signal provenant d'un circuit de minuterie.

5. Procédé selon l'une quelconque des revendications 1 à 4, comprenant en outre le calcul de manière sensiblement continue de nouveaux scores TSI calculés à l'aide d'une fenêtre glissante pour la série chronologique de fréquences cardiaques et la série chronologique de températures cutanées, et en option, la fenêtre glissante a une durée de 20 minutes ou 25 minutes.

6. Procédé selon l'une quelconque des revendications 1 à 5, comprenant en outre la génération d'un signal d'alerte par le processeur vers une alarme lorsque le score TSI dépasse un seuil d'alarme prédéterminé.

7. Système pour générer un indice de contrainte thermorégulatoire (TSI) pour un individu, le système comprenant : un processeur configuré pour

recevoir une série chronologique de mesures de température provenant d'un capteur de température fixé sur la peau de l'individu,

recevoir une série chronologique de fréquences cardiaques ou un signal de fréquence cardiaque provenant d'un capteur de fréquence cardiaque fixé sur l'individu, et

produire un score TSI pour l'individu en utilisant la série chronologique de fréquences cardiaques ou le signal de fréquence cardiaque provenant du capteur de fréquence cardiaque et la série chronologique de températures cutanées provenant du capteur de température en déterminant une complexité de fréquence cardiaque ou une fréquence cardiaque moyenne à partir de la série chronologique de fréquences cardiaques ou du signal de fréquence cardiaque,

déterminer une complexité de la température cutanée à partir de la série chronologique de températures cutanées, calculer un score TSI basé sur un rapport entre la complexité de la température cutanée et la complexité de la fréquence cardiaque, où le rapport est :

$$TSI = \frac{Tsk\_c}{HR\_c}$$

où TSI est le score de l'indice de contrainte thermorégulatrice, Tsk_c est la complexité de la température cutanée et HR_c est la complexité de la fréquence cardiaque, les complexités étant déterminées à l'aide de l'entropie approximative, et

tout score TSI égal ou inférieur à 1 indique que l'individu fait face à une contrainte thermique, tandis que tout score TSI supérieur à 1 indique que l'individu ne fait pas face à la contrainte thermique, et

produire un signal de sortie pour le score TSI ;

un affichage optionnel en communication électrique avec ledit processeur et configuré pour afficher le score TSI à partir du signal de sortie reçu dudit processeur ; et

un module de communication optionnel configuré pour communiquer le signal de sortie à un dispositif externe.

Measuring/receiving a time series of skin temperatures — 102

Measuring/receiving a time series of heart rates — 104

Determining a skin temperature complexity — 106

Determining a heart rate complexity — 108

Determining a TS score — 110

Providing the TS score — 112

**FIG. 1A**

```
┌─────────────────────────────────────────┐
│  Measuring/receiving a time series of skin │ ─── 102
│              temperatures                  │
└─────────────────────────────────────────┘
                    │
                    ▼
┌─────────────────────────────────────────┐
│  Measuring/receiving a time series of heart │ ─── 104
│                 rates                      │
└─────────────────────────────────────────┘
                    │
                    ▼
┌─────────────────────────────────────────┐
│  Determining a skin temperature complexity │ ─── 106
└─────────────────────────────────────────┘
                    │
                    ▼
┌─────────────────────────────────────────┐
│     Determining a heart rate complexity    │ ─── 108
└─────────────────────────────────────────┘
                    │
                    ▼
┌─────────────────────────────────────────┐
│           Determining a TS score           │ ─── 110
└─────────────────────────────────────────┘
                    │
                    ▼
┌─────────────────────────────────────────┐
│            Providing the TS score          │ ─── 112
└─────────────────────────────────────────┘
                    │
                    ▼
┌─────────────────────────────────────────┐
│  Performing an intervention when indicated by │ ─── 114
│                the TS score                │
└─────────────────────────────────────────┘
```

FIG. 1B

220

Heart Rate
Sensor

230

Temperature
Sensor

210

Processor

**FIG. 2**

220

Heart Rate
Sensor

230

Temperature
Sensor

210

Processor

340

Timer

**FIG. 3**

220

Heart Rate
Sensor

230

Temperature
Sensor

210

Processor

450

Alarm

**FIG. 4**

**FIG. 5**

FIG. 6

FIG. 7

FIG. 8A

FIG. 8B

FIG. 9A

FIG. 9B

**FIG. 9C**

**FIG. 9D**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63217600
- WO 2017181195 A1, Buller
- US 2019387982 A1

**Non-patent literature cited in the description**

- *Medical Surveillance Monthly Report*, 2019
- Heat Illness, Active Component. U.S. Armed Forces, 2020
- **BULLER MARK J et al.** Estimated and measured core temperature responses to high-intensity warm weather military training: implications for exertional heat illness risk assessment. *PHYSIOLOGICAL MEASUREMENT.*, 01 June 2020, vol. 41 (6)